(19) Europäisches Patentamt
European Patent Office
Office européen des brevets

(11) Veröffentlichungsnummer: **0 063 338**
**B1**

(12) **EUROPÄISCHE PATENTSCHRIFT**

(45) Veröffentlichungstag der Patentschrift: **13.02.85**

(51) Int. Cl.⁴: **C 07 D 307/935**, A 61 K 31/34 // C07C147/14

(21) Anmeldenummer: **82103073.1**

(22) Anmeldetag: **09.04.82**

# (54) 4-Oxo-PGI2-Derivate, Verfahren zu deren Herstellung sowie diese Verbindungen enthaltende pharmazeutische Präparate.

(30) Priorität: **14.04.81 HU 96681**

(43) Veröffentlichungstag der Anmeldung: **27.10.82 Patentblatt 82/43**

(45) Bekanntmachung des Hinweises auf die Patenterteilung: **13.02.85 Patentblatt 85/7**

(84) Benannte Vertragsstaaten: **BE CH DE FR GB IT LI NL SE**

(56) Entgegenhaltungen:
**GB - A - 2 016 007**
**US - A - 4 126 744**

**Die Akte enthält technische Angaben, die nach dem Eingang der Anmeldung eingereicht wurden und die nicht in dieser Patentschrift enthalten sind.**

(73) Patentinhaber: **CHINOIN Gyogyszer és Vegyészeti Termékek Gyára RT., To utca 1-5, H-1045 Budapest IV (HU)**

(72) Erfinder: **Galambos, Géza, Dr., Dipl.Ing.Chem., Lavotta u. 60, H-1104 Budapest (HU)**
Erfinder: **Simonidesz, Vilmos, Dipl.Ing.Chem., Fenyö u. 13, H-1016 Budapest (HU)**
Erfinder: **Székely, István, Dr., Dipl.Ing.Chem., Krajcár U. 6, H-2120 Dunakeszi (HU)**
Erfinder: **Ivanics, József, Dipl. Ing. Chem., Elelm u. 20, H-1045 Budapest (HU)**
Erfinder: **Kékesi, Krisztina, Dipl.Ing.Chem., Aprilis 4.-e ut 23, H-4029 Debrecen (HU)**
Erfinder: **Kovács, Gábor, Dr., Dipl.Ing.Chem., Róna Park 4, H-1142 Budapest (HU)**
Erfinder: **Stadler, István, Dr., Népstadion u. 18, H-1143 Budapest (HU)**
Erfinder: **Körmöczy, Péter, Dr., Uri u. 33, H-1014 Budapest (HU)**
Erfinder: **Horváth, Károly, Dr., Dipl.Ing.Chem., Szakasits A. u. 58/a., H-1115 Budapest (HU)**

(74) Vertreter: **Lotterhos, Hans Walter, Dr.-Ing., Lichtensteinstrasse 3, D-6000 Frankfurt am Main 1 (DE)**

EP 0 063 338 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft optisch aktive oder racemische 4-Oxo-$PGI_2$-Derivate der allgemeinen Formel I

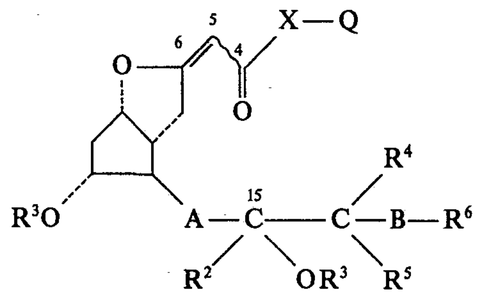

(I)

worin

Q eine $-COOR^1$, $-CH_2-OH$, $-COL^1$-Gruppe bedeutet,

$R^2$ ein Wasserstoffatom, ein Alkalimetall-, Erdalkalimetall-, Aluminium-Kation, ein Ammonium- oder Tris-hydroxymethyl-ammonium, $C_{1-6}$-Alkyl, $C_{3-10}$-Cycloalkyl, $C_{1-4}$-Alkyl, das durch unsubstituiertes oder durch Halogen, Phenyl, $C_{1-4}$-Alkyl oder durch halogensubstituiertes $C_{1-4}$-Alkyl substituiertes Phenyl substituiert ist, oder Phenyl, das durch Halogen, Phenyl, $C_{1-4}$-Alkyl oder halogensubstituiertes $C_{1-4}$-Alkyl substituiert sein kann,

$L^1$ Amino, $C_{1-6}$-Alkylamino oder Di-$C_{1-6}$-alkylamino bedeuten,

X $-CH_2-CH_2-$, $-CH=CH-$, $-CH_2-C(CH_3)_2-$, $-(CH_3)_2C-CH_2-$ oder Cyclopropylen,

A trans- oder cis$-CH=CH-$, $-C\equiv C-$, oder $-CH_2-CH_2-$,

$R^2$ ein Wasserstoffatom mit $\alpha$- oder $\beta$-Konfiguration, Methyl oder Äthyl,

$R^3$ ein Wasserstoffatom, $C_{1-4}$-Alkanoyl, Benzoyl, das durch Halogen, Phenyl, $C_{1-4}$-Alkyl oder halogensubstituiertes $C_{1-4}$-Alkyl substituiert sein kann, oder eine Tri-$C_{1-4}$-alkyl-silyl-Schutzgruppe,

$R^4$ und

$R^5$ unabhängig voneinander ein Wasserstoffatom oder $C_{1-4}$-Alkyl,

B Methylen, Sauerstoff oder $-NH-$ und

$R^6$ $C_{1-6}$-Alkyl,
Phenyl, das durch Halogen, Phenyl, $C_{1-4}$-Alkyl oder halogen substituiertes $C_{1-4}$-Alkyl substituiert sein kann, bedeuten.

Die neuen Verbindungen der Erfindung können als Metabolit der biologisch außerordentlich aktiven Arachidonsäure, als stabiles wirksames Analogen von Prostacyclin ($PGI_2$), betrachtet werden, das stärker als alle bisherigen Stoffe die Aggregation hemmt und über gefäßerweiternde Wirkung verfügt. Das $PGI_2$ hemmt selbst schon in Nanogramm-Menge die Aggregation von Blutplättchen bzw. löst die schon aggregierten Thromben auf. Wegen dieser einzigartigen pharmakologischen Wirkung kann es ein außerordentlich wertvolles Pharmakon bei der Heilung der Krankheit dieses Jahrhunderts, den Erkrankungen des Kreislaufs, in erster Linie der Thrombose, sein. Bis zum heutigen Tag sind zahlreiche Publikationen über die klinische Anwendung des Stoffes erschienen.

Das größte Problem bei der Anwendung von $PGI_2$ als Medikament bildet die außerordentliche Instabilität des Stoffes. In saurem oder neutralem Medium zerfällt es sofort zu dem mit der Form des Hemiketals in Gleichgewicht stehenden 6-Oxo-$PGF_1$. Das $PGI_2$ enthält eine außerordentlich reaktionsfähige Einheit mit Enolätherstruktur, die mit einer äußeren oder einer Protonenquelle innerhalb des Moleküls sofort in Reaktion tritt, so daß das $PGI_2$ in Form einer freien Säure nicht existenzfähig ist. Infolgedessen werden bei pharmakologischen und klinischen Versuchen die Salze und Ester eingesetzt.

Es sind schon zahlreiche Versuche unternommen worden, die Enolätherstruktur zu stabilisieren, die jedoch — wie gefunden wurde — für die pharmakologische Aktivität des $PGI_2$ wichtig ist.

Die aus der GB-A-2 016 007 bekannten $\omega$-Halogen-$PGI_2$-derivate, die die sehr reaktionsfähige Enolätherstruktureinheit mit der Doppelbindung in 5,6-Stellung enthalten, weisen zwar etwa die gleiche pharmakologische Aktivität auf wie das Prostacyclin aber auch dessen Instabilität. Sie werden zur Erhöhung der Stabilität in Cyclodextrin-Einschlußkomplexe übergeführt.

Die aus der US-PS 4 126 744 bekannten 4-Oxo-5,6-dihydroprostacycline oder 4-Oxo-$PGI_1$-Derivate, weisen zwar infolge der Hydrierung der 5,6-Doppelbindung die gewünschte Stabilität auf, sind aber in der pharmakologischen Aktivität dem Prostacyclin weit unterlegen. Die 4-Oxo gruppe hat auf die pharmakologische Aktivität praktisch keinen Einfluß.

Lediglich die 7-Oxo-$PGI_2$-Derivate der nicht vorveröffentlichten EP-A-131 426 weisen die gewünschte Stabilität neben ausgezeichneten pharmakologischen Eigenschaften auf. Dies ist damit zu erklären, daß durch den induktiven und konjugativen Effekt des Einbaus der Oxogruppe in 7-Stellung die Elektronendichte des Enoläthers, seine Nukleophilität, gesenkt wird, wodurch die Stabilität des Moleküls zunimmt.

Die bisher bekannten stabilen Prostacyclinanaloga waren unbefriedigend, da mit steigender Stabilität entweder deren pharmakologische Wirkung abnahm oder die Selektivität sank oder aber beides eintrat. Ziel der Erfindung war infolgedessen die Entwicklung von neuen Prostacyclinanaloga, die die Nachteile der bekannten Analoga nicht aufweisen. Überraschenderweise wurde gefunden, daß die neuen 4-Oxo-$GGI_2$-Derivate der allgemeinen Formel I über die für die praktische Anwendbarkeit notwendige Stabilität verfügen, ohne den starken pharmakologischen Aktivitätsverlust aufzuweisen.

In den 4-Oxo-$PGI_2$-Derivaten der Erfindung beeinflußt die Oxogruppe in 4-Stellung die Reaktionsfähigkeit des Enoläthers in der Weise, daß die Stabilität des Moleküls erhöht wird. In saurem und neutralem Medium weisen 4-Oxo-$PGI_2$ und dessen Analoga eine überraschende Stabilität auf — in 1n Salzsäure- und Essigsäuremedium können sie mehrere Stunden ohne nennenswerten Zerfall gehalten werden — da ihre Hauptabbauprodukte, das 6-Hydroxy-4-oxo-$PGI_1$ und dessen Derivate (die eine Einheit mit der $\beta$-Hydroxy-keton-Struktur aufweisen), zur ursprünglichen Form von 4-Oxo-$PGI_2$ zurückgebildet werden und sich das Gleichgewicht zugunsten letzterer verschiebt. Das 4-Oxo-$PGI_2$ und dessen Analoga können auch in Form der freien Säure isoliert und aufbewahrt werden, so können das 4-Oxo-$PGI_2$ und seine Derivate in saurem Medium als stabilste 9(0)-$PGI_2$-Analoga betrachtet werden.

Das 4-Oxo-$PGI_2$ der allgemeinen Formel I sowie dessen Analoga und Derivate hemmen durch Arachidonsäure oder Adenosinphosphat (ADP) oder Kollagen ausgelöste Blutplättchen-Aggregation, sie wirken auf den Kreislauf, auf die glatten Muskeln. Neu ist, daß sie eine cytoprotektive Wirkung aufweisen, auf die Muskeln der Bronchien, auf das gastrointestinale System wirken und über zahlreiche weitere wertvolle pharmakologische Wirkungen verfügen.

Die Verbindungen der allgemeinen Formel I hemmen als stabile Prostacyclinanaloga in einem an Blutplättchen, die aus Humanblut isoliert sind, reichen Plasma eine durch $1 \times 10^{-6}$ Mol/ml ADP ausgelöste Aggregation in einer Konzentration von 15—200 ng/ml ($IC_{50}$) (bestimmt nach der Methode von Born), was einer Wirkung von 1/10—1/100 $PGI_2$ entspricht.

Bei der Hemmung einer durch $10^{-4}$ Mol/ml ADP in einem an Kaninchen-Blutplättchen reichen Plasma ausgelösten Blutplättchenaggregation waren die 4-Oxo-$PGI_2$-Derivate der Erfindung den 4-Oxo-$PGI_1$-Derivaten der US-PS 4 126 744 stark überlegen. Während die Blutplättchenaggregation bereits mit 8 $\mu$g/ml des 4-Oxo-5-(Z)-$PGI_2$-methylesters der Erfindung gehemmt werden konnte, waren hierzu 25 $\mu$g/ml des 4-Oxo-$PGI_1$ der US-PS 4 126 744 erforderlich.

Diese neuen Prostacyclinanaloga verfügen — ähnlich wie Prostacyclin — über eine starke gefäßerweiternde Wirkung. Auf Grund der bei Messungen an mit Pentobarbitural narkotisierten Katzen erhaltenen hämodynamischen Parameter beträgt die Stärke der Wirkung 1/10—1/100 der Wirkung von $PGI_2$. Bei narkotisierten Hunden wird mit einer Infusion von 1—10 $\gamma$/kg/min erreicht, daß der Aorta-Blutdruck um 10—15% sinkt.

Die Verbindungen der allgemeinen Formel I senken den Ruhetonus von isolierten Kalbsherzkranzgefäßen; in zehnfacher Dosis ist ihre Wirkung identisch mit der von Prostacyclin. Sie führen die Erschlaffung der Trachea von Meerschweinchen und des isolierten Human-Bronchienpräparats herbei, die Stärke der Wirkung beträgt 1/25 der Wirkung von $PGI_2$. Weiterhin wirken sie auf das gastrointestinale System, sie hemmen die Sekretion von Magensäure.

Die Verbindungen der allgemeinen Formel I können als neue stabile Prostacyclinanaloga in der Human- und Tiermedizin zur Vorbeugung und Behandlung von unterschiedlichen Erkrankungen eingesetzt werden.

a) Die Applikation dieser Verbindungen senkt die Adhäsion der Blutplättchen, hemmt die Bildung von Thromben und löst bereits entstandene Thromben auf. So sind sie zur Vorbeugung und Behandlung von Myorkardinfarkten, postoperativer Thrombose und arteriosklerotischen Erkrankungen geeignet. Sie können auf dem Gebiet des extrakorporalen Kreislaufs allein oder kombiniert mit Heparin eingesetzt werden. Ihre Anwendung erfolgt oral, in Form von Tabletten oder Kapseln, weiterhin in Form von intravenösen und intramuskulären Injektionen oder Infusionen. Die tägliche Dosis beträgt je nach Form der Applikation, der Schwere der Erkrankung und dem Alter 0,05—50 mg/kg Körpergewicht.

b) Hemmung der Magensäurekretion

Die neuen Prostacyclinanaloga der Erfindung senken bei der Anwendung in der Human- und Tiermedizin die Bildung von Magensäure, sie sind infolgedessen zur Vorbeugung bzw. Therapie von Magengeschwüren geeignet. Ihre Applikation erfolgt intravenös, intramuskulär oder subkutan in Form einer Infusion. Die Dosis beträgt 0,1—20 $\gamma$/kg/min. Die orale Dosierung beträgt täglich 1—10 mg/kg Körpergewicht.

c) Bronchienerweiterung

Die neuen Prostacyclinanaloga der Erfindung sind zur Therapie von Asthma geeignet. Sie werden in Form von Tabletten, Kapseln oder Spray angewendet, die tägliche Dosis beträgt 0,01—5 mg/kg Körpergewicht. Sie können vorteilhaft mit anderen Antiasthmatika, z. B. Isoproterenol, Ephedrin, Xanthin-Derivaten und Corticosteroiden, kombiniert werden.

d) Weiterhin können sie zur Therapie von verschiedenen Hautkrankheiten, z. B. Psoriasis, unterschiedlicher aspezifischer und spezifischer Dermatitis, allergischen Ausschlägen, verwendet werden. Hierbei werden sie in Form von Lösungen, Salben und Sprays lokal verwendet, der Wirkstoffgehalt in den Präparaten beträgt 0,5—2%.

Die Erfindung betrifft auch ein neues Verfahren zur Herstellung der optisch aktiven und racemischen 4-Oxo-$PGI_2$-Derivate der allgemeinen Formel I, wonach man

a) Verbindungen der allgemeinen Formel III

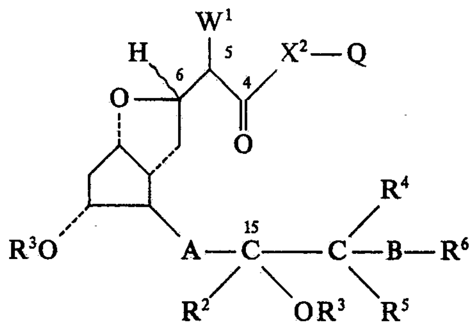

(III)

worin

Q, $R^1$, $R^2$, $R^3$, $R^4$, $R^5$, $R^6$, $L^1$, A und B die oben angegebene Bedeutung haben,
$W^1$ eine Alkyl-thio-, Aryl-thio-, substituierte Aryl-thio- oder Aryl-seleno-Gruppe und
$X^2$ $-CH_2-CH_2-$, $-CH=CH-$, $-CH_2-C(CH_3)_2-$, $-C(CH_3)_2-CH_2-$,

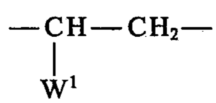 oder 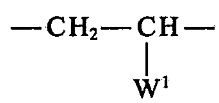

bedeuten,

durch Behandeln mit einer organischen oder anorganischen Perverbindung in einem inerten Lösungsmittel bei einer Temperatur zwischen —78° C und 50° C oxydiert und dann aus den erhaltenen 5-substituierten 4-Oxo-$PGI_1$-Derivaten der allgemeinen Formel II

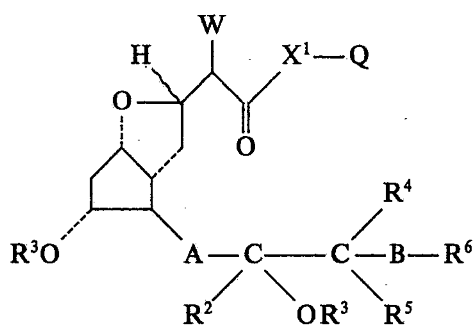

(II)

worin

Q, $R^1$, $R^2$, $R^3$, $R^4$, $R^5$, $R^6$, $L^1$, A und B die oben angegebene Bedeutung haben,
W eine Alkyl-sulfinyl-, Aryl-sulfinyl-, substituierte Aryl-sulfinyl- oder Aryl-selenyl-Gruppe und
$X^1$ $-CH_2-CH_2-$, $-CH=CH-$, $-CH_2-C(CH_3)_2-$, $-C(CH_3)_2-CH_2$,

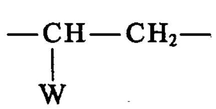 oder 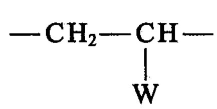

bedeuten,

das W-H-Molekül durch Erhitzen im Temperaturbereich zwischen 0 und 200° C oder durch Behandlung mit einer Base eliminiert, oder

b) Verbindungen der allgemeinen Formel IV

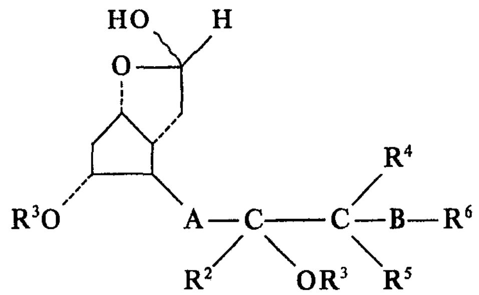

(IV)

worin
$R^2$, $R^3$, $R^4$, $R^5$, $R^6$, A und B die oben angegebene Bedeutung haben, in einem Lösungsmittel in Gegenwart eines Knoevenagel-Katalysators mit Verbindungen der allgemeinen Formel V

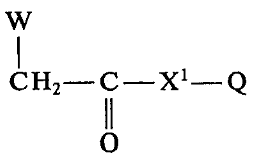

(V)

worin
Q, $R^1$, $X^1$ und W die oben angegebene Bedeutung haben,
kondensiert und in den erhaltenen Verbindungen der allgemeinen Formel I — gewünschtenfalls — durch Verseifung, Hydrolyse, Salzbildung, die Einführung einer Schutzgruppe die vorhandenen Substituenten in andere Substituenten der Verbindungen der allgemeinen Formel I überführt.

Gemäß Verfahrensvariante a) können für die Oxydation der 5-Thio- oder Seleno-verbindungen der allgemeinen Formel III zu den entsprechenden 5-Sulfinyl- oder Selenyl-verbindungen der allgemeinen Formel II Wasserstoffperoxyd, organische Persäuren, m-Chlor-perbenzoesäure, Natrium-meta-perjodat sowie sonstige für diese Oxydation geeignete Reaktionsmittel verwendet werden (E. Block, Reactions of OrganoSulfur Compounds, Academic Press Inc. New York, San Francisco, London, 1978). Die Temperatur der Reaktionsgemische kann innerhalb weiter Grenzen — von —78°C bis +50°C — schwanken. Als Lösungsmittel können alle inerten organischen Lösungsmittel, vorteilhaft chlorierte aliphatische Kohlenwasserstoffe, und Lösungsmittel vom Äthertyp, eingesetzt werden.

Die Eliminierungsreaktion der Verfahrensvariante a) kann thermisch oder in Gegenwart einer Base erfolgen. Die Temperatur der Reaktionsgemische kann zwischen 0—200°C schwanken. Als Lösungsmittel können aromatische Kohlenwasserstoffe, Benzol, Toluol, Xylol, halogenierte aliphatische und aromatische Kohlenwasserstoffe, z. B. Dichloräthan oder Chlorbenzol, aprotische dipolare Lösungsmittel, z. B. Dimethylformamid, Dimethylsulfoxyd, Hexamethylphosphorsäure-triamid, weiterhin tertiäre organische Basen, Pyridin, substituierte Pyridine, Trialkylamine, 1,5-Diazabicyclo[5,4,0]undec-5-en (DBU) oder 1,5-Diazabicyclo[4,3,0]non-5-en (DBN) eingesetzt werden. Als Base können organische und anorganische Basen, vorteilhaft tertiäre aliphatische und aromatische Amine, Träthylamin, Pyridin, DBU, DBN, Alkali-alkoxyde, Kaliumcarbonat usw. verwendet werden.

Die Oxydation der Aryl-seleno-verbindungen der allgemeinen Formel III wird zweckmäßig bei einer Temperatur zwischen —78° C und 0° C und die Eliminierungsreaktion bei 0—100° C durchgeführt.

Die Verbindungen der allgemeinen Formel III können aus bicyclischen Hemiacetalderivaten der allgemeinen Formel IV mit 5-substituierten 4-Oxo-valeriansäure-Derivaten, die eine aktivierte Methylen-Gruppe enthalten, in einer Verfahrensstufe mittels der Knoevenagel-Kondensation mit nachfolgender Michael-Addition hergestellt werden. Wenn der 5-Substituent der an der Knoevenagel-Reaktion teilnehmenden Valeriansäure-Derivate der allgemeinen Formel V so gewählt wird, daß er für eine Eliminierungsreaktion geeignet ist, so können 4-Oxo-$PGI_2$ und dessen Analoga sowie Derivate aus den Verbindungen der allgemeinen Formel IV in einer Verfahrensstufe hergestellt werden.

Die Verbindungen der allgemeinen Formel IV sind aus der Literatur bekannt (JACS, 91, 5675, (1969)) bzw. sie können wie dort beschrieben hergestellt werden.

Gemäß Verfahrensvariante b) werden die Verbindungen der allgemeinen Formel IV mit Verbindungen der allgemeinen Formel V in einem Lösungsmittel in Gegenwart eines Knoevenagel-Katalysators kondensiert. Die Temperatur des Reaktionsgemisches kann innerhalb weiter Grenzen — von 80—200°C — schwanken. Als Lösungsmittel werden aromatische und halogenierte aromatische Kohlenwasserstoffe, vorteilhaft Toluol, Xylol oder Chlorbenzol, bei einer dem Siedepunkt des Lösungsmittels entsprechenden Temperatur verwendet. Weiterhin können aprotische dipolare Lösungsmittel, z. B. Dimethylformamid, Dimethylsulfoxyd, Hexamethyl-phosphorsäure-triamid, eingesetzt werden. Als Katalysator der Reaktionen können alle bekannten Katalysator-Typen der Knoevenagel-Reaktion, vorteilhaft sekundäre Amine, Piperidin oder Morpholin, oder deren mit organischen Säuren gebildeten

Salze verwendet werden.

Die nach den beiden Methoden erhaltenen Verbindungen der allgemeinen Formel I können in die 5(Z)- und 5(E)-Isomeren getrennt werden.

Der Aufbau des 4-Oxo-$PGI_2$-Moleküls über die Knoevenagel-Kondensation unter Verwendung der entsprechenden Sulfinyl- oder Selenyl-Derivate ist neu. Gemäß der US-PS 4 126 744 erfolgt der Aufbau des 4-Oxo-$PGI_1$-Derivats über die entsprechenden Phosphorane.

In der Beschreibung sind unter $C_{1-4}$-Alkyl Methyl-, Äthyl-, n- und i-Propyl-, n-, i-, sec- und tert.-Butyl und unter $C_{1-4}$-Alkanoyl die, die aus den den oben angegebenen $C_{1-4}$-Alkylen entsprechenden Alkanen abgeleitet werden können, das sind Formyl, Acetyl, Propionyl und Butyryl, zu verstehen. Die $C_{1-6}$-Alkyle umfassen über die $C_{1-4}$-Alkyle hinaus auch unterschiedliche Pentyl- und Hexylgruppen. Unter Aryl ist in der Beschreibung Phenyl zu verstehen, das gegebenenfalls an beliebiger Stelle ein- oder mehrmals durch Halogen, durch eine Phenylgruppe, eine $C_{1-4}$-Alkylgruppe oder eine durch Halogen substituierte $C_{1-4}$-Alkylgruppe substituiert sein kann. Die Aralkylgruppe kann eine $C_{1-4}$-Alkylgruppe sein, die durch eine der oben definierten Arylgruppen substituiert ist.

Characteristische Vertreter für $C_{3-10}$-Cycloalkylgruppen sind die Cyclopropyl-, Cyclopentyl-, Cyclohexyl-, Cycloheptyl-, Cyclooctyl-, Cyclononyl- und Cyclodecyl-Gruppe.

Wenn in der Beschreibung nicht anders angegeben, ist unter einem pharmakologisch verträglichem Kation das Äquivalent von Ionen mit der positiven Ladung 1, 2 oder 3 zu verstehen, die im lebenden Organismus bei den für die Verbindungen der Erfindung angegebenen Dosen keine unerwünschte Nebenwirkungen hervorrufen. Beispiele für solche Kationen sind die Ionen der Alkalimetalle, das Natrium-, Kalium- und Lithiumion, die Ionen der Erdalkalimetalle, z. B. das Calcium- und Magnesiumion, das Aluminion, das Ammoniumion, und die aus unterschiedlichen organischen Aminen ableitbaren ein- oder mehrwertigen Ammoniumionen, z. B. das Tris-hydroxymethylammoniumion.

Die Alkylgruppen der Tri-$C_{1-4}$-alkyl-silyl-Gruppen können identisch oder unterschiedlich sein.

Als besonders vorteilhaft haben sich folgende Verbindungen der Erfindung erwiesen:

4-Oxo-$PGI_2$-methylester,
4-Oxo-16,16-dimethyl-$PGI_2$-methylester,
4-Oxo-$PGI_2$-methylester-11,15-diacetat,
4-Oxo-5Z-$PGI_2$,
4-Oxo-13,14-didehydro-20-äthyl-$PGI_2$-methylester,
2,2-Dimethyl-4-oxo-16-phenyl-17,18,19,20-tetranor-$PGI_2$-methylester,
2-Decarboxy-2-hydroxy-methyl-4-oxo-$PGI_2$,
4-Oxo-20-methyl-$PGI_2$-methylester,
4-Oxo-20-äthyl-$PGI_2$-methylester,
2,2-Dimethyl-4-oxo-$PGI_2$-methylester,
$\Delta^2$-4-Oxo-$PGI_2$-methylester,
$\Delta^2$-4-Oxo-16,16-dimethyl-$PGI_2$-methylester,
$\Delta^2$-13,14-Didehydro-4-oxo-$PGI_2$-methylester,
4-Oxo-13,14-didehydro-$PGI_2$-methylester,
4-Oxo-13,14-didehydro-20-methyl-$PGI_2$-methylester,
2,2-Dimethyl-4-oxo-13,14-didehydro-$PGI_2$-methylester,
4-Oxo-16-phenoxy-17,18,19, 20-tetranor-$PGI_2$-methylester,
4-Oxo-16-(m-chlor-phenoxy)-17,18,19,20-tetranor-$PGI_2$-methylester,
4-Oxo-16-(m-trifluor-methyl-phenoxy)-17,18,19,20-tetranor-$PGI_2$-methylester,
15-Epi-4-oxo-$PGI_2$-methylester,
15-Epi-4-oxo-13,14-didehydro-$PGI_2$-methylester,
15-Epi-4-oxo-16-phenoxy-17,18,19,20-tetranor-$PGI_2$-methylester,
15-Epi-$\Delta^2$-4-oxo-$PGI_2$-methylester,
15-Epi-2,2-dimethyl-4-oxo-$PGI_2$-methylester,
15-Epi-4-oxo-20-methyl-$PGI_2$-methylester,
15-Epi-4-oxo-20-äthyl-$PGI_2$-methylester und
15-Epi-4-oxo-13,14-didehydro-20-äthyl-$PGI_2$-methylester.

Weitere Einzelheiten der Erfindung werden anhand folgender Beispiele veranschaulicht, ohne die Erfindung zu beschränken.

## Beispiel 1

### 5-Phenyl-sulfinyl-4-oxo-$PGI_1$-methylester

(Formel II: W = Phenyl—So—, X = $-CH_2-CH_2-$, Q = $COOR^1$, $R^1$ = Methyl, A = trans—CH=CH—, $R^2$ = Wasserstoff mit $\beta$-Konfiguration, $R^3$, $R^4$ und $R^5$ = Wasserstoff, B = Methylen, $R^6$ = Propyl)

1,60 g (3,3 mM) 5-Phenyl-thio-4-oxo-$PGI_1$-methylester (Formel III: $W^1$ = Phenyl—S—, die übrigen Substituenten: wie oben angegeben) werden in 80 ml Dichlormethan gelöst und unter Rühren bei 0° C mit 690 mg (3,6 mM) 90% Wirkstoff enthaltender m-Chlor-perbenzoesäure versetzt. Die Reaktion dauert 5 Minuten (Kontrolle durch Dünnschichtchromatographie mit Äthylacetat—Aceton (Verhältnis 3 : 1, als Eluent). Dem Reaktionsgemisch setzt man bei 0° C 10 ml 10%ige Natriumhydrogen-sulfitlösung zu und rührt 2—3 Minuten. Dann wird das erhaltene Gemisch mit 250 ml Äthylacetat versetzt und nacheinander mit 40 ml Wasser, 40 ml gesättigter Natriumhydrogencarbonatlösung, 40 ml Wasser und schließlich mit 30 ml gesättigter wäßriger Natriumchloridlösung gewaschen. Nach dem Trocknen über Magnesiumsulfat wird filtriert und das Lösungsmittel abdestilliert. Mit Hilfe von kurzer Säulenchromatographie (Eluent: Äthylacetat — Aceton 3 : 1) werden 1,60 g (96,8%) der im Titel genannten Verbindung in Form weißer Kristalle erhalten.

$R_f$: 0,24 mit dem Gemisch von Äthylacetat — Aceton im Verhältnis 3 : 1 eluiert,
IR (KBr): 1710, 1750 $cm^{-1}$ (C=O),
$^1H$ NMR ($CDCl_3$):
$\delta$ 7,55 (5 H, m, aromatisch),
5,5 (2 H, m, —CH=CH—),
3,5—4,7 (5 H, m),
3,60 ppm (3 H, s, $COOCH_3$).

## Beispiel 2

### 4-Oxo-$PGI_2$-methylester

(Formel I: die übrigen Substituenten entsprechen der im Titel von Beispiel 1 genannten Verbindung.)

20 ml Dimethylformamidlösung von 552 mg (1,09 mM) 5-Phenyl-sulfinyl-4-oxo-$PGI_1$-methylester erhitzt man unter Rühren 2—3 Stunden auf 145—150° C und setzt, dem Reaktionsgemisch 150 ml Äthylacetat zu. Dann wird es nacheinander 3 × mit 25 ml Wasser, mit 25 ml gesättigter wäßriger Natriumchloridlösung gewaschen, über Magnesiumsulfat getrocknet, filtriert und vom Lösungsmittel durch Destillation befreit. Durch kurze Säulenchromatographie auf Silicagel mit dem Gemisch Äthylacetat—Aceton (Verhältnis 4 : 1) werden 249 mg (60%) 4-Oxo-5[E]-$PGI_2$-methylester und 37,4 mg (9%) 4-Oxo-5[Z]-$PGI_2$-methylester als im Titel genannte Verbindung in Form von farblosen Kristallen erhalten.

4-Oxo-5[E]-$PGI_2$-methylester: Schmelzpunkt: 89—92° C.
$R_f$: 0,37 mit dem Gemisch von Äthylacetat — Aceton (Verhältnis 3 : 1) eluiert,
$^1H$ NMR ($CDCl_3$):
$\delta$ 5,80 (1 H, breit s, C=CH—C=O),
5,6 (2 H, m),
4,77 (1 H, m),
3,5—4,2 (5 H, m, einschließlich 3,68, s, $COOCH_3$),
$^1H$ NMR ($C_6D_6$):
$\delta$ 5,95 (1 H, breit s, C=CH—C=O),
5,5 (2 H, m),
4,35 (1 H, m),
4,1 (1 H, m),
3,7 (1 H, m),
3,45 ppm (3 H, s, $COOCH_3$).
4-Oxo-5[Z]-$PGI_2$-methylester:
$R_f$: 0,25 mit dem Gemisch von Äthylacetat — Aceton (Verhältnis 3 : 1) eluiert,
$^1H$ NMR ($C_6D_6$):
$\delta$ 5,5 (2 H, m, —CH=CH—),
5,22 (1 H, s, C=CH—C=O),
4,4 (1 H, m),
4,15 (1 H, m),

3,71 (1 H, m),
3,47 (3 H, s, $COOCH_3$).

## Beispiel 3

### 5-Phenyl-sulfinyl-4-oxo-$PGI_1$-methylester—11,15-diacetat

(Formel II: $R^3$ = Acetyl, die übrigen Substituenten entsprechen der im Titel von Beispiel 1 genannten Verbindung.)

110 mg (0,19 mM) 5-Phenyl-thio-4-oxo-$PGI_1$-methylester-11,15-diacetat (Formel III: $W^1$ = Phenyl—S, $R^3$ = Acetyl, die übrigen Substituenten: wie oben angegeben) löst man in 5 ml Dichlormethan und setzt der Lösung bei 0°C 40,4 mg (0,21 mM) 90% Wirkstoff enthaltende m-Chlor-perbenzoesäure zu. Die Reaktion dauert 5 Minuten. Dann setzt man 5 ml 10%ige Natriumsulfitlösung zu und extrahiert mit 50 ml Äthylacetat. Die organische Phase wird nacheinander mit 5 ml Wasser, 5 ml gesättigter wäßriger Natriumhydrogen-carbonatlösung, 5 ml Wasser und schließlich 5 ml gesättigter Natriumchloridlösung gewaschen. Sie wird dann über Magnesiumsulfat getrocknet, filtriert und vom Lösungsmittel durch Destillation befreit. Mit kurzer Säulenchromatographie unter Verwendung des Gemisches Äthylacetat — Hexan (Verhältnis 1 : 1) werden 101 mg (89%) der im Titel genannten Verbindung in Form eines farblosen Öls isoliert.

$R_f$: 0,20 mit dem Gemisch von Äthylacetat — Hexan (Verhältnis 1 : 1) eluiert,
$^1H$ NMR ($CDCl_3$:
$\delta$7,6 (5 H, m),
5,5 (2 H, m),
3,9—5,3 (5 H, m),
3,67 (3 H, s),
2,05 (3 H, s),
2,00 ppm (3 H, s).

## Beispiel 4

### 5-Phenyl-sulfinyl-4-oxo-valeriansäure-methylester

(Formel V: W = Phenyl—SO, X = $-CH_2-CH_2-$, Q = Methoxy-carbonyl)

20 ml Dichlormethanlösung von 500 mg (2,1 mM) 5-Phenyl-thio-4-oxo-valeriansäure-methylester werden bei 0°C 443 mg (2,56 mM) 90%ige m-Chlorperbenzoesäure zugesetzt. Die Reaktion dauert 5 Minuten. Dann werden 5 ml 10%ige Natriumhydrogen-sulfitlösung zugesetzt und nach 5 Minuten Rühren 60 ml Äthylacetat zugegeben. Die organische Phase wird dann nacheinander mit 10 ml Wasser, 10 ml Natriumhydrogen-carbonatlösung, 10 ml Wasser, 10 ml gesättigter wäßriger Natriumchloridlösung gewaschen, über Magnesiumsulfat getrocknet, filtriert und vom Lösungsmittel durch Destillation befreit. Mit Hilfe von kurzer Säulenchromatographie und unter Verwendung des Gemisches Äthylacetat — Hexan (Verhältnis 1 : 1) als Eluent werden 515 mg (96,4%) der im Titel genannten Verbindung in Form von weißen Kristallen isoliert.

Schmelzpunkt: 50°C,
$R_f$: 0,19 mit dem Gemisch von Äthylacetat — Hexan (Verhältnis 4 : 1) eluiert,
$^1H$ NMR ($CDCl_3$):
$\delta$7,4—7,8 (5 H, m),
3,92 (2 H, s),
3,67 (3 H, s),
2,4—2,9 ppm (4 H, m).

## Beispiel 5

### 4-Oxo-$PGI_2$-methylester

92 mg (0,34 mM) 3$\alpha$,$\beta$-Hydroxy-6$\beta$-(3 S-hydroxy-oct-1E-enyl)-7$\alpha$-hydroxy-2-oxabicyclo[3,3,0]octan löst man in 5 ml Xylol, setzt 260 mg (1,02 mM) 5-Phenylsulfinyl-4-oxo-valeriansäure-methylester und 34 µl (0,34 µM) Piperidin zu und erhitzt das Reaktionsgemisch 6—8 Stunden auf 140—150°C. Das Rohprodukt wird durch kurze Säulenchromatographie gereinigt, als Eluent wird das Lösungsmittelgemisch Äthylacetat — Aceton (Verhältnis 4 : 1) verwendet. Es werden 55,1 mg (42%) 4-Oxo-5(E)-$PGI_2$-methylester und 21,4 mg (16,5%) 4-Oxo-5(Z)-$PGI_2$-methylester isoliert, dessen physikalische Konstanten identisch mit denen der Verbindung von Beispiel 2 sind.

## Beispiel 6

### 5-Phenyl-sulfinyl-16,16-dimethyl-4-oxo-$PGI_1$-methylester

(Formel II: $R^5$ und $R^4$ = Methyl, die übrigen Substituenten entsprechen der im Titel von Beispiel 1 genannten Verbindung.)

160 mg (0,31 mM) 5-Phenyl-thio-16,16-dimethyl-4-oxo-$PGI_1$-methylester (Formel III: $W^1$ = Phenyl-S, $R^4$ und $R^5$ = Methyl, die übrigen Substituenten: wie oben angegeben) löst man in 8 ml Dichlormethan und setzt der Lösung bei 0°C 69 mg (0,36 mM) 90% Wirkstoff enthaltende m-Chlor-perbenzoesäure zu. Nach 5 Minuten Rühren wird dem Reaktionsgemisch 1 ml 10%ige Natriumhydrogen-sulfitlösung zugesetzt und 2—3 Minuten bei 0°C gerührt. Das Gemisch wird dann mit 30 ml Äthylacetat verdünnt, mit 5 ml Wasser, 2× mit 5 ml gesättigter wäßriger Natriumhydrogen-carbonatlösung, 5 ml Wasser und schließlich mit 5 ml gesättigter wäßriger Natriumchloridlösung gewaschen. Sodann wird das Reaktionsgemisch über Magnesiumsulfat getrocknet und vom Lösungsmittel durch Destillation befreit. Die Chromatographie erfolgt auf 15 g Silicagel mit Äthylacetat. Es werden 158 mg (95%) der im Titel genannten Verbindung in Form eines farblosen Öls erhalten.

$R_f$: 0,4 mit Äthylacetat eluiert,
$^1H$ NMR ($CDCl_3$):
$\delta$7,48 (5 H, m),
5,52 (2 H, m),
3,5—4,7 (5 H, m),
3,63 (3 H, s, $COOCH_3$).

## Beispiel 7

### 16,17-Dimethyl-4-oxo-$PGI_2$-methylester

116 mg (0,2 mM) 5-Phenyl-sulfinyl-16,16-dimethyl-4-oxo-$PGI_1$-methylester löst man in 2 ml Dimethylformamid und hält die Lösung 3 Stunden auf 140—150°C. Die Lösung wird mit 20 ml Äthylacetat verdünnt, mit 5 ml Wasser und 5 ml gesättigter wäßriger Natriumchloridlösung gewaschen, über Magnesiumsulfat getrocknet und vom Lösungsmittel durch Destillation befreit. Die Chromatographie erfolgt auf 10 g Silicagel mit Äthylacetat. Es werden 48 mg (54,5%) 16,16-Dimethyl-4-oxo-5(E)-$PGI_2$-methylester und 13 mg (14,7%) 16,16-Dimethyl-4-oxo-5(Z)-$PGI_2$-methylester als die im Titel genannten Verbindungen in Form von farblosem Öl gewonnen.

16,16-Dimethyl-4-oxo-5(E)-$PGI_2$-methylester:
$R_f$: 0,63 mit Äthylacetat eluiert,
$^1H$ NMR ($CDCl_3$):
$\delta$5,83 (1 H, breit s, C=CH—C=O),
5,58 (2 H, m, —CH=CH—),
3,63 ppm (3 H, s, $COOCH_3$).
16,16-Dimethyl-4-oxo-5(Z)-$PGI_2$-methylester:
$R_f$: 0,48 mit Äthylacetat eluiert,
$^1H$ NMR ($C_6D_6$):
$\delta$5,5 (2 H, m, —CH=CH—),
5,26 (1 H, s, C=CH—C=O),
3,49 ppm (3 H, s, $COOCH_3$).

## Beispiel 8

### 4-Oxo-$PGI_2$-methylester-11,15-diacetat

64 mg (0,1 mM) 5-Phenyl-sulfinyl-4-oxo-$PGI_1$-methylester-11,15-diacetat (Verbindung des Beispiels 3) werden in 3 ml Dimethylformamid gelöst und bei 110°C 10 Stunden lang gerührt. Die Lösung wird mit Äthylacetat verdünnt, zunächst mit Wasser, dann mit gesättigter Salzlösung gewaschen und über Magnesiumsulfat getrocknet. Kurze Säulenchromatographie mit dem Gemisch Äthylacetat — Hexan

(Verhältnis 1 : 1). Es werden 39,3 mg (53,8%) 5[E]-Isomer und 8,8 mg (12,0%) 5[Z]-Isomer als im Titel genannte Verbindung in Form eines farblosen Öls erhalten.

4-Oxo-5(E)-$PGI_2$-methylester-11,15-diacetat:
$R_f$: 0,37 mit dem Gemisch von Äthylacetat — Hexan (Verhältnis 1 : 1) eluiert,
$^1H$ NMR ($C_6D_6$):
$\delta$5,92 (1 H, s),
5,3—5,6 (3 H, m),
3,46, 1,95, 1,97 ppm (3 H, s).
4-Oxo-5(Z)-$PGI_2$-methylester-11,15-diacetat:
$R_f$: 0,24 mit dem Gemisch Äthylacetat — Hexan (Verhältnis 1 : 1) eluiert,
$^1H$ NMR ($C_6D_6$):
$\delta$5,2—5,6 (4 H, m),
2,05 (3 H, s),
1,95 ppm (3 H, s).

## Beispiel 9

### 4-Oxo-5[Z]-$PGI_2$

190 mg (0,5 mM) 4-Oxo-5[Z]-$PGI_2$-methylester werden in 2 ml Methanol gelöst und unter Zugabe von 1 ml 1 n Natriumhydroxidlösung 5 Stunden bei Raumtemperatur gerührt. Sodann wird mit Wasser verdünnt, 3× mit 5 ml Äthylacetat extrahiert, mit Natriumhydrogensulfatlösung der pH-Wert der wäßrigen Phase auf 1—2 eingestellt, 3× mit 5 ml Äthylacetat wird extrahiert, mit Wasser gewaschen, über Magnesiumsulfat getrocknet und vom Lösungsmittel durch Destillation befreit. Es werden 170 mg (92%) der im Titel genannten Verbindung in Form eines farblosen Öls gewonnen, das bei 0° C langsam erstarrt.

$R_f$: 0,17 mit dem Gemisch von Aceton—Äthylacetat (Verhältnis 3 : 1) eluiert.

## Beispiel 10

### 13,14-Didehydro-20-äthyl-4-oxo-$PGI_2$-methylester

(Formel I: A = Äthinylen, $R^6$ = Pentyl, die übrigen Substituenten entsprechen der im Titel von Beispiel 1 genannten Verbindung.)

300 mg (1 mM) 3$\alpha$,$\beta$-Hydroxy-6$\beta$-(3S-hydroxy-dec-1-inyl)-7$\alpha$-hydroxy-2-oxabicyclo[3,3,0]octan werden in 15 ml Xylol gelöst und 760 mg (3 mM) 5-Phenyl-sulfinyl-4-oxo-valeriansäure-methylester sowie 0,1 ml Piperidin zugesetzt. Das Reaktionsgemisch wird nach Einsetzen eines Wasserabscheideaufsatzes 6—8 Stunden erhitzt. Das Rohprodukt wird durch kurze Säulenchromatographie gereinigt, wobei als Eluent das Lösungsmittelgemisch Äthylacetat — Aceton (Verhältnis 6 : 1) angewendet wird. Es werden 220 mg (55%) 13,14-Didehydro-20-äthyl-oxo-5[E]-$PGI_2$-methylester und 60 mg (15%) 13,14-Didehydro-20-äthyl-4-oxo-5[Z]-$PGI_2$-methylester als im Titel genannte Verbindung in Form eines farblosen Öls erhalten.

13,14-Didehydro-20-äthyl-4-oxo-5[E]-$PGI_2$-methylester:
$R_f$: 0,46 mit dem Gemisch Äthylacetat — Aceton (Verhältnis 3 : 1) eluiert,
$^1H$ NMR ($C_6D_6$):
$\delta$5,92 (1 H, s, C=CH—C=O),
4,39 (2 H, m),
4,13 (1 H, m),
3,76 (1 H, m),
3,45 (3 H, s, $COOCH_3$).
13,14-Didehydro-20-äthyl-4-oxo-5[Z]-$PGI_2$-methylester:
$^1H$ NMR ($C_6D_6$):
$\delta$5,24 (1 H, s, C≡CH—C=O),
4,43 (1 H, m),
4,15 (1 H, m),
3,8 (1 H, m),
3,48 (3 H, s, $COOCH_3$).

Beispiel 11

2,2-Dimethyl-16-phenoxy-17,18,19,20-tetranor-4-oxo-$PGI_2$-methylester

(Formel I: X = $-CH_2-C(CH_3)_2-$, B = Sauerstoff, $R^6$ = Phenyl, die übrigen Substituenten entsprechen der im Titel von Beispiel 1 genannten Verbindung.)

570 mg (2 mM) 3α,β-Hydroxy-6β-(3 S-hydroxy-4-phenoxy-but-1 E-enyl)-7α-hydroxy-2-oxabicyclo[3,3,0]octan werden in 25 ml Xylol gelöst und 1,7 g (6 mM) 2,2-Dimethyl-5-phenyl-sulfinyl-4-oxo-valeriansäuremethylester sowie 0,2 ml Piperidin zugesetzt. Das Reaktionsgemisch wird nach Einsetzen eines Wasserabscheideaufsatzes 8 Stunden erhitzt. Das Rohprodukt wird durch kurze Säulenchromatographie und dem Gemisch Äthylacetat — Aceton (Verhältnis 5 : 1) gereinigt. Es werden 466 mg (55%) 2,2-Dimethyl-16-phenoxy-17,18,19,20-tetranor-4-oxo-5[E]-$PGI_2$-methylester und 150 mg (18%) 2,2-dimethyl-16-phenoxy-17,18,19,20-tetranor-4-oxo-5[Z]-$PGI_2$-methylester als im Titel genannte Verbindung in Form eines farblosen Öls erhalten.

2,2-Dimethyl-16-phenoxy-17,18,19,20-tetranor-4-oxo-5[E]-$PGI_2$-methylester:
$R_f$: 0,41 mit dem Gemisch Äthylacetat — Aceton (Verhältnis 4 : 1) eluiert,
$^1H$ NMR ($CDCl_3$):
δ5,8 (1 H, s, C=CH—C=O),
7,1—7,4 (5 H, m, aromatisch),
5,6 (2 H, m, —CH=CH),
4,7—3,9 (5 H, m, CH—O),
3,67 (3 H, s, $COOCH_3$).

2,2-Dimethyl-16-phenoxy-17,18,19,20-tetranor-4-oxo-5[Z]-$PGI_2$-methylester:
$R_f$: 0,27 mit dem Gemisch Äthylacetat — Aceton (Verhältnis 4 : 1) eluiert,
$^1H$ NMR ($C_6D_6$):
δ7,1—7,4 (5 H, m, aromatisch),
5,5 (2 H, m, —CH=CH),
5,2 (1 H, s, C=CH—C=O),
4,45—3,71 (5 H, m, CH—O),
3,45 (3 H, s, $COOCH_3$).

Beispiel 12

2-Decarboxy-2-hydroxy-methyl-4-oxo-$PGI_2$

(Formel I: Q = Hydroxy-methyl, die übrigen Substituenten entsprechen der im Titel von Beispiel 1 genannten Verbindung.)

270 mg (1 mM) 3α,β-Hydroxy-6β-(3S-hydroxy-oct-1E-enyl)-7α-hydroxy-2-oxabicyclo[3,3,0]octan werden in 10 ml Xylol gelöst, dann 720 mg (3 mM) 5-Phenylsulfinyl-4-oxo-pentanol und 0,1 ml Piperidin zugesetzt. Das Reaktionsgemisch wird 6 Stunden auf 140—150°C gehalten. Das Rohprodukt wird durch kurze Säulenchromatographie gereinigt, als Eluent wird das Lösungsmittelgemisch Äthylacetat — Aceton (Verhältnis 3 : 1) verwendet. Es werden 173 mg (52%) 2-Decarboxy-2-hydroxy-methyl-4-oxo-5[E]-$PGI_2$ und 63 mg (18%) 2-Decarboxy-2-hydroxy-methyl-4-oxo-5[Z]-$PGI_2$ als im Titel genannte Verbindung in Form eines farblosen Öls erhalten.

2-Decarboxy-2-hydroxy-methyl-4-oxo-5[E]-$PGI_2$:
$R_f$: 0,39 mit dem Gemisch Äthylacetat — Aceton (Verhältnis 3 : 1) eluiert,
$^1H$ NMR ($CDCl_3$):
δ5,86 (1 H, s, C=CH—C=O),
5,55 (2 H, m, —CH=CH—),
4,6—3,7 (5 H, m, CH—O),
0,9 (3 H, t, $-CH_3$).

2-Decarboxy-2-hydroxy-methyl-4-oxo-5[Z]-$PGI_2$:
$R_f$: 0,27 mit dem Gemisch Äthylacetat — Aceton (Verhältnis 3 : 1) eluiert,
$^1H$ NMR ($C_6D_6$):
δ5,5 (2 H, m, —CH=CH—),
5,18 (1 H, s, C=CH—C=O),
4,7—3,7 (5 H, m, CH—O),
0,92 (3 H, t, $-CH_3$).

Die folgenden 4-Oxo-$PGI_2$-Analoga wurden aus den entsprechend substituierten Ausgangsstoffen

nach den Beispielen 1—12 in Form der Isomere von 5(E) und 5(Z) hergestellt:

20-methyl-4-oxo-$PGI_2$-methylester
$R_f$: 5(E): 0,377 5(Z): 0,25 Äthylacetat — Aceton 3 : 1,
20-Äthyl-4-oxo-$PGI_2$-methylester
$R_f$: 5(E): 0,38 5(Z): 0,25 Äthylacetat — Aceton 3 : 1,
2,2-Dimethyl-4-oxo-$PGI_2$-methylester
$R_f$: 5(E): 0,34 5(Z): 0,23 Äthylacetat — Aceton 3 : 1,
$\Delta^2$-4-Oxo-$PGI_2$-methylester
$R_f$: 5(E): 0,36 5(Z): 0,23 Äthylacetat — Aceton 3 : 1,
16,16-Dimethyl-$\Delta^2$-4-oxo-$PGI_2$-methylester
$R_f$: 5(E): 0,43 5(Z): 0,35 Äthylacetat — Aceton 2 : 1,
13,14-Didehydro-$\Delta^2$-4-oxo-$PGI_2$-methylester
$R_f$: 5(E): 0,38 5(Z): 0,24 Äthylacetat,
13,14-Didehydro-4-oxo-$PGI_2$-methylester
$R_f$: 5(E): 0,42 5(Z): 0,30 Äthylacetat,
13,14-Didehydro-20-methyl-4-oxo-$PGI_2$-methylester
$R_f$: 5(E): 0,40 5(Z): 0,30 Äthylacetat,
2,2-Dimethyl-13,14-didehydro-4-oxo-$PGI_2$-methylester
$R_f$: 5(E): 0,38 5(Z): 0,28 Äthylacetat,
16-Phenoxy-17,18,19,20-tetranor-4-oxo-$PGI_2$-methylester
$R_f$: 5(E): 0,53 5(Z): 0,46 Äthylacetat — Aceton 1 : 1,
16-(m-Chlor-phenoxy)-17,18,19,20-tetranor-4-oxo-$PGI_2$-methylester
$R_f$: 5(E): 0,53 5(Z): 0,45 Äthylacetat — Aceton 1 : 1,
16-(m-Trifluor-methyl-phenoxy)-17,18,19,20-tetranor-4-oxo-$PGI_2$-methylester
$R_f$: 5(E): 0,58 5(Z): 0,46 Äthylacetat — Aceton 1 : 1,
15-Epi-4-oxo-$PGI_2$-methylester
$R_f$: 5(E): 0,37 5(Z): 0,26 Äthylacetat,
15-Epi-13,14-didehydro-4-oxo-$PGI_2$-methylester
$R_f$: 5(E): 0,40 5(Z): 0,30 Äthylacetat,
15-Epi-16-phenoxy-17,18,19,20-tetranor-4-oxo-$PGI_2$-methylester
$R_f$: 5(E): 0,53 5(Z): 0,46 Äthylacetat — Aceton 1 : 1,
15-Epi-$\Delta^2$-4-oxo-$PGI_2$-methylester
$R_f$: 5(E): 0,38 5(Z): 0,24 Äthylacetat — Aceton 3 : 1,
15-Epi-2,2-dimethyl-4-oxo-$PGI_2$-methylester
$R_f$: 5(E): 0,36 5(Z): 0,23 Äthylacetat — Aceton 3 : 1,
15-Epi-20-methyl-4-oxo-$PGI_2$-methylester
$R_f$: 5(E): 0,36 5(Z): 0,26 Äthylacetat — Aceton 3 : 1,
15-Epi-20-äthyl-4-oxo-$PGI_2$-methylester
$R_f$: 5(E): 0,38 5(Z): 0,25 Äthylacetat — Aceton 3 : 1,
15-Epi-13,14-dihehydro-20-äthyl-4-oxo-$PGI_2$-methylester
$R_f$: 5(E): 0,40 5(Z): 0,31 Äthylacetat.

Außerdem wurden aus den oben angegebenen Verbindungen durch Hydrolyse Säuren und Salze und aus letzteren Säureamide hergestellt. Durch die Reduktion der Säuren können 2-Decarboxy-2-hydroxy-methyl-Derivate gewonnen werden.

Die in der Beschreibung aufgeführten $R_f$-Werte wurden auf einer MERCK Kieselgel 60 $F_{254}$-Platte bestimmt.

Die in Verfahren a. als Ausgangsmaterial verwendeten Verbindungen der allgemeinen Formel III können ähnlich wie der nachfolgend berschriebene 5-Phenyl-thio-4-oxo-$PGI_1$-methylester hergestellt werden.

5-Phenyl-thio-4-oxo-$PGI_1$-methylester

(Formel III: $W^1 = C_6H_5$-S, die übrigen Substituenten entsprechen der im Titel von Beispiel 1 genannten Verbindung)

Das 920 mg (3,4 mM) 3$\alpha$,$\beta$-Hydroxy-6$\beta$-(3S-hydroxy-oct-1E-enyl)-7$\alpha$-hydroxy-2-oxabicyclo[3,3,0]octan, 1,64 g (6,88 mM) 5-Phenyl-sulfenyl-4-oxo-valeriansäuremethylester, 5 ml Benzol und 7 ml 0,5 molare benzolhaltige Piperidiniumacetatlösung enthaltende Reaktionsgemisch wird unter Anwendung eines Wasserabscheideaufsatzes 60 Stunden erhitzt. Nach Beendigung der Reaktion (Kontrolle durch Dünnschichtchromatographie) wird das Gemisch nach Zusatz von 100 ml Äthylacetat 2× mit 10 ml Wasser, 10 ml gesättigter wäßriger Natriumchloridlösung gewaschen, über Magnesiumsulfat getrocknet und dann vom Lösungsmittel durch Destillation befreit. Das Rohprodukt wird auf 30 g Silicagel bei einem Überdruck von 1,5 bar mit Äthylacetat als Eluent der Chromatographie unterworfen.

Es werden 1,4 g (84%) der im Titel genannten Verbindung in Form eines farblosen Öls erhalten.

$R_f$: 0,46 mit dem Gemisch Äthylacetat — Aceton (Verhältnis 1 : 1) eluiert,
IR (Film): 1750, 1710 $cm^{-1}$ (C=O),
$^1H$ NMR ($CDCl_3$):
$\delta$7,4—7,6 (3 H, m, aromatisch),
7,0—7,2 (2 H, m, aromatisch),
5,6—5,7 (2 H, m, —C=CH—),
3,4—4,6 (7 H, 2 H austauschbar durch $D_2O$),
3,45 ppm (3 H, s, $COOCH_3$).

**Patentansprüche**

1. Optisch aktive und racemische 4-Oxo-$PGI_2$-Derivate der allgemeinen Formel I

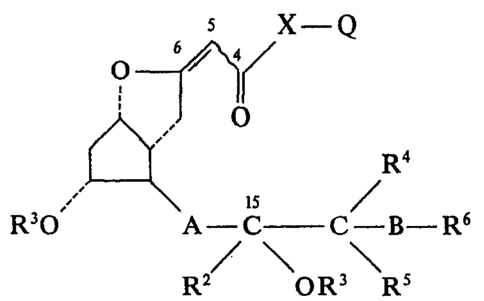

(I)

worin

Q eine $-COOR^1$, $-CH_2-OH$, $-COL^1$-Gruppe bedeutet, in der
$R^1$ ein Wasserstoffatom, eine Alkalimetall-, Erdalkalimetall-, Aluminium-Kation, ein Ammonium- oder Tris-hydroxymethyl-ammonium, $C_{1-6}$-Alkyl, $C_{3-10}$-Cycloalkyl, $C_{1-4}$-Alkyl, das durch unsubstituiertes oder durch Halogen, Phenyl, $C_{1-4}$-Alkyl oder durch halogensubstituiertes $C_{1-4}$-Alkyl substituiertes Phenyl substituiert ist, oder Phenyl, das durch Halogen, Phenyl, $C_{1-4}$-Alkyl oder halogensubstituiertes $C_{1-4}$-Alkyl substituiert sein kann,
$L^1$ Amino, $C_{1-6}$-Alkylamino oder Di-$C_{1-6}$-alkylamino bedeuten,
X $-CH_2-CH_2-$, $-CH=CH-$, $-CH_2-C(CH_3)_2-$, $-(CH_3)_2C-CH_2$- oder Cyclopropylen,
A trans oder cis $-CH=CH-$, $-C\equiv C-$, oder $-CH_2-CH_2-$,
$R^2$ ein Wasserstoffatom mit $\alpha$- oder $\beta$-Konfiguration, Methyl oder Äthyl,
$R^3$ ein Wasserstoffatom, $C_{1-4}$-Alkanoyl, Benzoyl, das durch Halogen, Phenyl, $C_{1-4}$-Alkyl oder halogensubstituiertes $C_{1-4}$-Alkyl substituiert sein kann, oder eine Tri-$C_{1-4}$-alkyl-silyl-Schutzgruppe,
$R^4$ und
$R^5$ unabhängig voneinander ein Wasserstoffatom oder $C_{1-4}$-Alkyl,
B Methylen, Sauerstoff oder —NH— und
$R^6$ $C_{1-6}$-Alkyl, Phenyl, das durch Halogen, Phenyl, $C_{1-4}$-Alkyl oder halogensubstituiertes $C_{1-4}$-Alkyl substituiert sein kann, bedeuten.

2. 4-Oxo-$PGI_2$-methylester.
3. 4-Oxo-13,14-didehydro-$PGI_2$-methylester.
4. 4-Oxo-13,14-didehydro-20-methyl-$PGI_2$-methylester.
5. 4-Oxo-5(Z)-$PGI_2$.
6. Verfahren zur Herstellung von optisch aktiven und racemischen 4-Oxo-$PGI_2$-Derivaten der allgemeinen Formel I

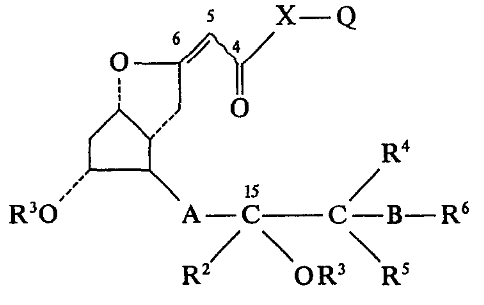

(I)

worin
Q, $R^1$, $R^2$, $R^3$, $R^4$, $R^5$, $R^6$, X, $L^1$, A und B die oben angegebene Bedeutung haben, dadurch gekennzeichnet, daß man

a) Verbindungen der allgemeinen Formel III

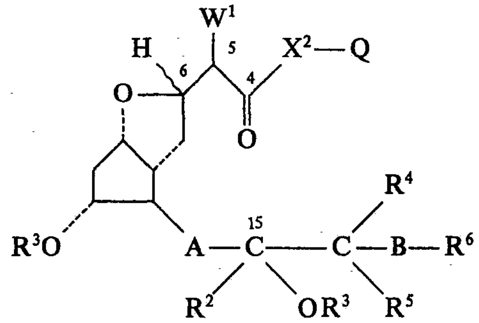

(III)

worin
Q, $R^1$, $R^2$, $R^3$, $R^4$, $R^5$, $R^6$, $L^1$, A und B die oben angegebene Bedeutung haben,
$W^1$ eine Alkyl-thio-, Aryl-thio-, substituierte Aryl-thio- oder Aryl-seleno-Gruppe und
$X^2$ $-CH_2-CH_2-$, $-CH=CH-$, $-CH_2-C(CH_3)_2-$, $H-C(CH_3)_2-CH_2-$,

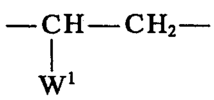 oder 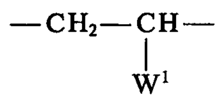

bedeuten,

durch Behandeln mit einer organischen oder anorganischen Perverbindung in einem inerten Lösungsmittel bei der Temperatur zwischen $-78°C$ und $+50°C$ oxydiert und dann aus den erhaltenen 5-substituierten 4-Oxo-$PGI_1$-Derivaten der allgemeinen Formel II

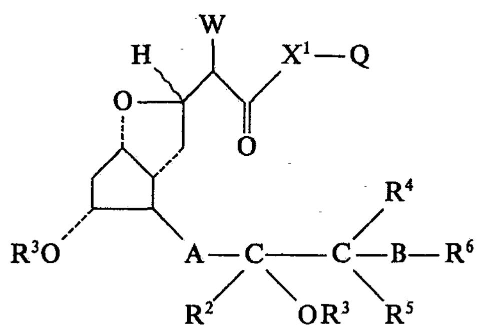

(II)

worin
Q, $R^1$, $R^2$, $R^3$, $R^4$, $R^5$, $R^6$, $L^1$, A und B die oben angegebene Bedeutung haben,
W eine Alkyl-sulfinyl-, Aryl-sulfinyl-, substituierte Aryl-sulfinyl- oder Aryl-selenyl-Gruppe und
$X^1$ $-CH_2-CH_2-$, $-CH=CH-$, $-CH_2-C(CH_3)_2-$, $-C(CH_3)_2-CH_2-$,

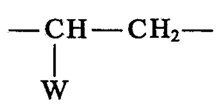 oder 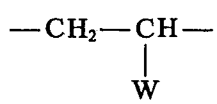

bedeuten,

das W-H-Molekül durch Erhitzen im Temperaturbereich zwischen 0 und 200° C oder durch Behandlung mit einer Base eliminiert, oder

b) Verbindungen der allgemeinen Formel IV

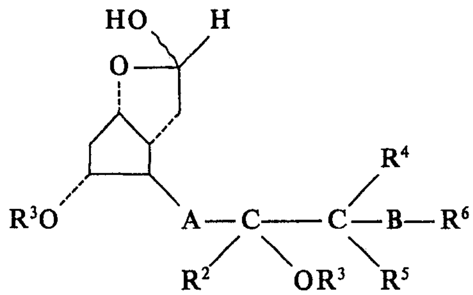

(IV)

worin
$R^2$, $R^3$, $R^4$, $R^5$, $R^6$, A und B die oben angegebene Bedeutung haben, in einem Lösungsmittel in Gegenwart eines Knoevenagel-Katalysators mit Verbindungen der allgemeinen Formel V

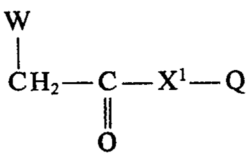

(V)

worin
Q, $R^1$, $X^1$ und W die oben angegebene Bedeutung haben,
kondensiert und in den erhaltenen Verbindungen der allgemeinen Formel I — gewünschtenfalls — durch Verseifung, Hydrolyse, Salzbildung, die Einführung einer Schutzgruppe die vorhandenen Substituenten in andere Substituenten der Verbindungen der allgemeinen Formel I überführt.

7. Verfahren nach Anspruch 6, dadurch gekennzeichnet, daß man zur Oxydation der Verfahrensvariante a) als Perverbindung Wasserstoffperoxyd, Natrium-meta-perjodat oder m-Chlorperbenzoesäure verwendet.

8. Verfahren nach Anspruch 6 oder 7, dadurch gekennzeichnet, daß man die Eliminierungsreaktion der Verfahrensvariante a) bei einer Temperatur von 80 bis 200°C in Gegenwart von aromatischen Kohlenwasserstoffen, halogenierten aromatischen oder aliphatischen Kohlenwasserstoffen oder aprotischen polaren Lösungsmitteln durchführt.

9. Verfahren nach Anspruch 6, dadurch gekennzeichnet, daß man die Kondensation der Verfahrensvariante b) bei einer Temperatur von 80 bis 200°C in Gegenwart von sekundären Aminen, deren mit organischen Säuren gebildeten Salzen, und in Gegenwart von aromatischen und halogenierten aromatischen Kohlenwasserstoffen, Dimethyl-formamid, Dimethyl-sulfoxyd oder Hexamethyl-phosphorsäuretriamid, als Lösungsmittel durchführt.

10. Verfahren nach Anspruch 6, dadurch gekennzeichnet, daß man aus Verbindungen der allgemeinen Formel I, die in 1-Stellung einen Esterrest aufweisen, durch Behandeln mit einer Base oder einer Säure die entsprechende freie Säure herstellt, die gewünschtenfalls in ein Salz übergeführt werden kann.

11. Verfahren nach Anspruch 1 bis 10, dadurch gekennzeichnet, daß man die in Form eines Gemisches von 5E-5Z-Isomeren erhaltenen Verbindungen der allgemeinen Formel I durch Chromatographie in die 5E- und 5Z-Isomeren trennt.

12. Pharmazeutische Präparate, die als Wirkstoff eine oder mehrere der 4-Oxo-$PGI_2$-Derivate der Ansprüche 1 bis 5 enthalten.

**Claims**

1. Optically active and racemic 4-oxo-$PGI_2$ derivatives of general formula I

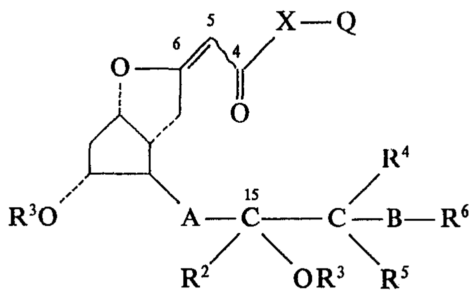

(I)

wherein

Q represents a $-COOR^1$, $-CH_2-OH$, $-COL^1$ group,
wherein
$R^1$ represents a hydrogen atom, an alkali metal, alkaline earth metal or aluminium cation, an ammonium or tris-hydroxymethyl-ammonium ion, $C_{1-6}$ alkyl, $C_{3-10}$ cycloalkyl, $C_{1-4}$ alkyl which is substituted by unsubstituted phenyl or by phenyl substituted by halogen, phenyl, $C_{1-4}$ alkyl or by halogen-substituted $C_{1-4}$ alkyl, or phenyl which may be substituted by halogen, phenyl, $C_{1-4}$ alkyl or halogen-substituted $C_{1-4}$ alkyl,
$L^1$ represents amino, $C_{1-6}$ alkylamino or Di-$C_{1-6}$-alkylamino,
X represents $-CH_2-CH_2-$, $-CH=CH-$, $-CH_2-C(CH_3)_2-$, $-(CH_3)_2C-CH_2-$ or cyclopropylene,
A represents trans- or cis$-CH=CH-$, $-C\equiv C-$, or $-CH_2-CH_2-$,
$R^2$ represents a hydrogen atom with $\alpha$- or $\beta$-configuration, methyl or ethyl,
$R^3$ represents a hydrogen atom, $C_{1-4}$-alkanoyl, benzoyl which may be substituted by halogen, phenyl, $C_{1-4}$ alkyl or halogen substituted $C_{1-4}$ alkyl, or a tri-$C_{1-4}$-alkylsilyl protecting group,
$R^4$ and
$R^5$, independently of each other, represent a hydrogen atom or $C_{1-4}$ alkyl,
B represents methylene, oxygen or $-NH-$ and
$R^6$ represents $C_{1-6}$ alkyl, phenyl which may be substituted by halogen, phenyl, $C_{1-4}$ alkyl or halogen-substituted $C_{1-4}$ alkyl.

2. 4-Oxo-$PGI_2$-methyl ester.
3. 4-Oxo-13,14-didehydro-$PGI_2$-methyl ester.
4. 4-Oxo-13,14-didehydro-20-methyl-$PGI_2$-methyl ester.
5. 4-Oxo-5(Z)-$PGI_2$.
6. Process for preparing optically active and racemic 4-oxo-$PGI_2$ derivatives of general formula I

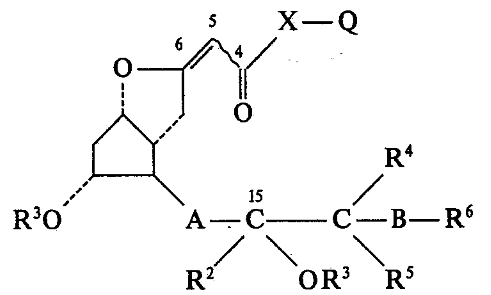

(I)

wherein
Q, $R^1$, $R^2$, $R^3$, $R^4$, $R^5$, $R^6$, X, $L^1$, A and B are as hereinbefore defined,
characterised in that
a) compounds of general formula III

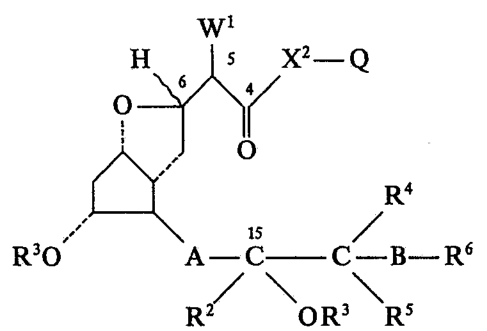

(III)

wherein
Q, $R^1$, $R^2$, $R^3$, $R^4$, $R^5$, $R^6$, $L^1$, A and B are as hereinbefore defined,
$W^1$ represents an alkyl-thio, aryl-thio, substituted aryl-thio or aryl-seleno group and
$X^2$ represents $-CH_2-CH_2-$, $-CH=CH-$, $-CH_2-C(CH_3)_2-$, $-C(CH_3)_2-CH_2-$,

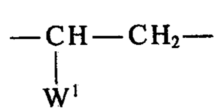 or 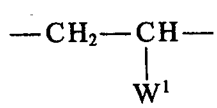

are oxidised by treating them with an organic or inorganic percompound in an inert solvent at a

temperature of between −78°C and +50°C and then the W—H molecule is eliminated from the resulting 5-substituted 4-Oxo-$PGI_1$ derivatives of general formula II

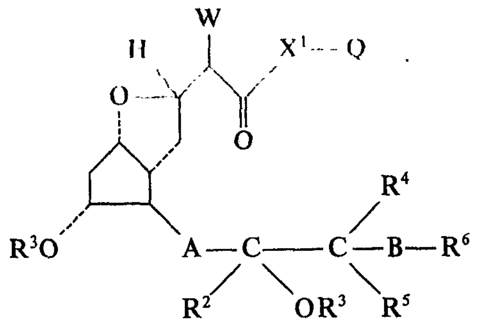

(II)

wherein
Q, $R^1$, $R^2$, $R^3$, $R^4$, $R^5$, $R^6$, $L^1$, A and B are as hereinbefore defined,
W represents an alkyl-suphinyl, aryl-sulphinyl, substituted aryl-sulphinyl or aryl-selenyl group and
$X^1$ represents $-CH_2-CH_2-$, $-CH=CH-$, $-CH_2-C(CH_3)_2-$, $-C(CH_3)_2-CH_2-$,

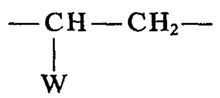 or 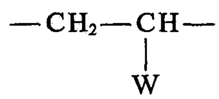

by heating in a temperature range of between 0 and 200° C or by treatment with a base, or
b) Compounds of general formula IV

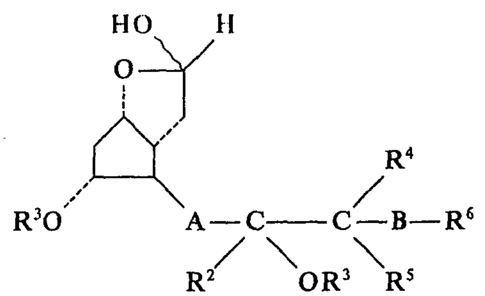

(IV)

wherein
$R^2$, $R^3$, $R^4$, $R^5$, $R^6$, A and B are as hereinbefore defined, are condensed in a solvent in the presence of a Knoevenagel catalyst with compounds of general formula V

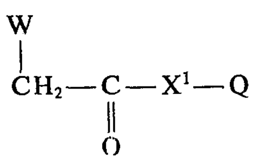

(V)

wherein
Q, $R^1$, $X^1$ and W are as hereinbefore defined,
and in the resulting compounds of general formula I the substituents present are, if desired, converted into other substituents of the compounds of general formula I by saponification, hydrolysis, salt formation or the introduction of a protecting group.

7. Process as claimed in claim 6, characterised in that for the oxidation in process variant a) hydrogen peroxide, sodium metaperiodate or m-chloro-perbenzoic acid is used as the percompound.

8. Process as claimed in claim 6 or claim 7, characterised in that the reaction of elimination in process variant a) is carried out at a temperature of 80 to 200°C in the presence of aromatic hydrocarbons, halogenated aromatic or aliphatic hydrocarbons or aprotic polar solvents.

9. Process as claimed in claim 6, characterised in that the condensation in process variant b) is carried out at a temperature of from 80 to 200°C in the presence of secondary amines, the salts thereof formed with organic acids, and in the presence of aromatic and halogenated aromatic hydrocarbons, dimethylformamide, dimethylsulphoxide or hexamethyl phosphoric acid triamide as the solvent.

10. Process as claimed in claim 6, characterised in that the corresponding free acid is prepared from compounds of general formula I having an ester group in the 1 position by treatment with a base or an acid and this free acid may if desired be converted into a salt.

11. Process as claimed in claims 1 to 10, characterised in that the compounds of general formula I obtained in the form of a mixture of 5E and 5Z isomers are separated into the 5E and 5Z isomers by chromatography.

12. Pharmaceutical preparations containing as active substance one or more of the 4-oxo-$PGI_2$ derivatives of claims 1 to 5.

**Revendications**

1. Dérivés énantiomères et racémiques de 4-oxo-$PGI_2$ de formule I

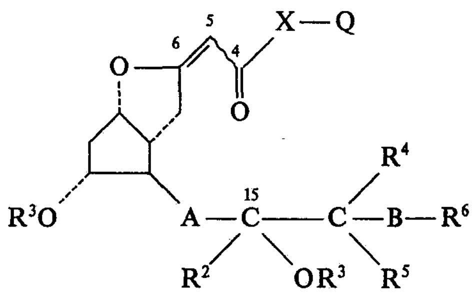

(I)

dans laquelle

Q représente un groupe $-COOR^1$, $-CH_2-OH$, $-COL^1$, dans lequel:

$R^1$ représente un atome d'hydrogène, un cation de métal alcalin, de métal alcalino-terreux, d'aluminium, un ion ammonium ou trishydroxyméthylammonium, un groupe alkyle en $C_1-C_6$, cycloalkyle en $C_3-C_{10}$, alkyle en $C_1-C_4$ substitué par au moins un groupe phényle lui-même non substitué ou substitué par au moins un radical halogéno, phényle, alkyle en $C_1-C_4$ ou halogéno-alkyle en $C_1-C_4$, ou bien un groupe phényle non substitué ou substitué par au moins un radical halogéno, phényle, alkyle en $C_1-C_4$ ou halogéno alkyle en $C_1-C_4$,

$L^1$ représente un groupe amino, alkylamino en $C_1-C_6$ ou di-(alkyle en-$C_1-C_6$)-amino,

X représente un groupe $-CH_2-CH_2-$, $-CH=CH-$, $-CH_2-C(CH_3)_2-$, $-(CH_3)_2C-CH_2-$ ou cyclopropylène,

A représente un groupe trans- ou cis-$CH=CH-$, $-C\equiv C-$ ou $-CH_2-CH_2-$,

$R^2$ représente un atome d'hydrogène en configuration $\alpha$ ou $\beta$, un groupe méthyle ou éthyle,

$R^3$ représente un atome d'hydrogène, un groupe alcanoyle $C_1-C_4$, benzoyle non substitué ou substitué par au moins un radical halogéno, phényle, alkyle en $C_1-C_4$ ou halogéno-alkyle en $C_1-C_4$, ou un groupe protecteur tri-(alkyle en $C_1-C_4$)-silyle,

$R^4$ et $R^5$ représentent, indépendamment l'un de l'autre, un atome d'hydrogène ou un groupe alkyle en $C_1-C_4$,

B représente un groupe méthylène, l'oxygène ou un groupe $-NH-$ et

$R^6$ représente un groupe alkyle en $C_1-C_6$, phényle non substitué on substitué par au moins un radical halogéno, phényle, alkyle en $C_1-C_4$ ou halogéno-alkyle en $C_1-C_4$.

2. L'ester méthylique de 4-oxo-$PGI_2$.

3. L'ester méthylique de 4-oxo-13,14-dideshydro-$PGI_2$.

4. L'ester méthylique de 4-oxo-13,14-dideshydro-20-méthyl-$PGI_2$.

5. Le 4-oxo-5(Z)-$PGI_2$.

6. Procédé de préparation de dérivés énantiomères et racémiques de 4-oxo-$PGI_2$ de formule générale

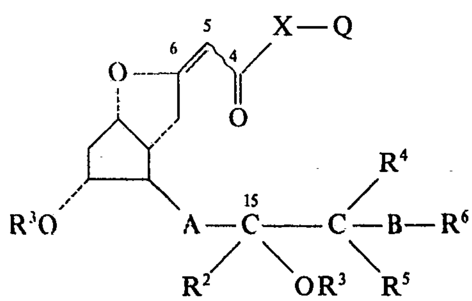

(I)

dans laquelle:

Q, $R^1$, $R^2$, $R^3$, $R^4$, $R^5$, $R^6$, X, $L^1$, A et B ont les significations indiquées ci-dessus, caractérisé en ce que:

a) on oxyde par traitement à l'aide d'un composé peroxydé organique ou minéral dans un solvant inerte à une température de −78° C à +50° C des composés de formule générale III

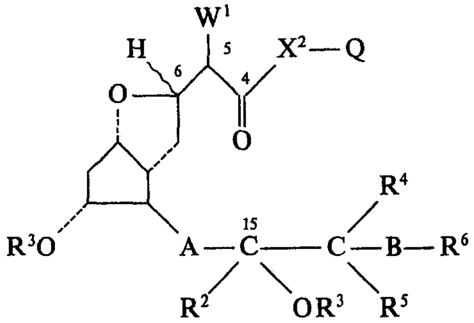

(III)

dans laquelle:
Q, $R^1$, $R^2$, $R^3$, $R^4$, $R^5$, $R^6$, $L^1$, A et B ont les significations indiquées ci-dessus,
$W^1$ représente un groupe alkylthio, arylthio, arylthio substitué ou arylséléno, et
$X^2$ représente un groupe $-CH_2-CH_2-$, $-CH=CH-$, $-CH_2-C(CH_3)_2-$, $-C(CH_3)_2-CH_2-$,

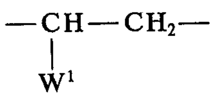
ou
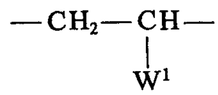

puis, à partir des dérivés de 4-oxo-$PGI_1$ substitués en position 5 ainsi obtenus et répondant à la formule générale II

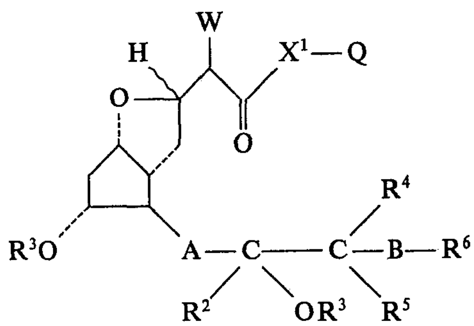

(II)

dans laquelle:
Q, $R^1$, $R^2$, $R^3$, $R^4$, $R^5$, $R^6$, $L^1$, A et B ont les significations indiquées ci-dessus,
W représente un groupe alkylsulfinyle, arylsulfinyle, arylsulfinyle substitué ou arylsélényle, et
$X^1$ représente un groupe $-CH_2-CH_2-$, $-CH=CH-$, $-CH_2-C(CH_3)_2-$, $-C(CH_3)_2-CH_2-$,

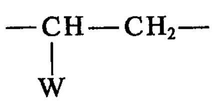
ou
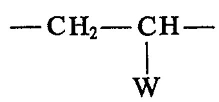

on élimine la molécule W−H par chauffage dans un intervalle de température de 0 à 200° C ou par traitement à l'aide d'une base, ou bien

b) on condense des composés de formule générale IV

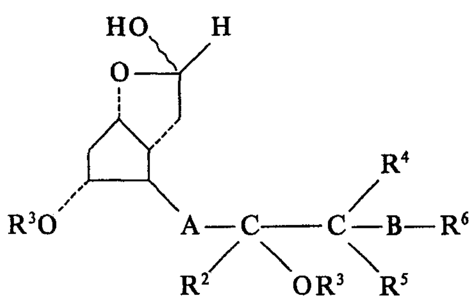

(IV)

dans laquelle:

$R^2$, $R^3$, $R^4$, $R^5$, $R^6$, A et B ont les significations indiquées ci-dessus, dans un solvant, en présence d'un catalyseur de Knoevenagel, avec des composés de formule générale V

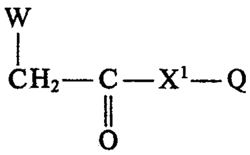

(V)

dans laquelle:

Q, $R^1$, $X^1$ et W ont les significations indiquées ci-dessus,

et dans les composés obtenus répondant à la formule générale I — le cas échéant — on convertit les substituants présents en d'autres substituants des composés de formule générale I par saponification, hydrolyse, salification, introduction d'un groupe protecteur.

7. Procédé selon la revendication 6, caractérisé en ce que, pour l'oxydation de la variante opératoire a), on utilise en tant que composé peroxydé le peroxyde d'hydrogène, le métaperiodate de sodium ou l'acide m-chloroperbenzoïque.

8. Procédé selon la revendication 6 ou 7, caractérisé en ce que la réaction d'élimination de la variante opératoire a) est effectuée à une température de 80 à 200°C en présence d'hydrocarbures aromatiques, d'hydrocarbures aromatiques ou aliphatiques halogénés ou de solvants polaires aprotoniques.

9. Procédé selon la revendication 6, caractérisé en ce que la condensation de la variante opératoire b) est effectuée à une température de 80 à 200°C en présence d'amines secondaires, de leurs sels formés avec des acides organiques, et en présence d'hydrocarbures aromatiques et aromatiques halogénés, de diméthylformamide, de diméthylsulfoxyde ou d'hexaméthylphosphorotriamide servant de solvants.

10. Procédé selon la revendication 6, caractérisé en ce que, à partir des composés de formule générale I portant un reste d'ester en position 1, on prépare, par traitement à l'aide d'une base ou d'un acide, l'acide libre correspondant qu'on peut convertir si on le désire en un sel.

11. Procédé selon les revendications 1 à 10, caractérisé en ce que l'on sépare les composés de formule générale I obtenus à l'état de mélange d'isomères 5E-5Z, par chromatographie, en les isomères 5E et 5Z.

12. Compositions pharmaceutiques contenant en tant que substance active un ou plusieurs des dérivés de 4-oxo-$PGI_2$ des revendications 1 à 5.